# EUROPEAN PATENT APPLICATION

(11) **EP 4 008 345 A1**
(43) Date of publication of application: **08.06.2022**
(21) Application number: 20211703.2
(22) Date of filing: 03.12.2020
(51) Int. Cl.: A61K 39/395, C07K 16/28

(54) **NOVEL FORMULATIONS FOR ANTIBODIES**

(71) Applicant: Hexal AG, 83607 Holzkirchen (DE)
(72) Inventor: ZUPANCIC, Urban, 1526 Ljubljana (SI); ZULA, Ales, 1526 Ljubljana (SI); SINK, Roman, 1526 Ljubljana (SI); BERGER, Lukas, 6336 Langkampfen (AT); VIERTLER, Martin, 6336 Langkampfen (AT); WINKLER, Ulrike, 6336 Langkampfen (AT); ROZMAN, Petrusa, 1526 Ljubljana (SI); SAJEVIC, Tamara, 1526 Ljubljana (SI); SUSTERI, Ana, 1526 Ljubljana (SI); BONINA, Matjaz, 1526 Ljubljana (SI); ANKO, Maja, 1526 Ljubljana (SI)
(74) Representative: Rückerl, Florian

(57) **Abstract**

The present invention relates in general to a stable, preferably liquid, pharmaceutical formulation comprising an antibody, a buffer, a surfactant, a stabilizer selected from the group consisting of sugars and sugar alcohols, and further optionally a chelator and/or oxygen-scavenger. Furthermore, the present invention relates to an article of manufacture comprising a container and a stable pharmaceutical formulation of the invention.

SEQUENCE LISTING

<110>  Hexal AG

<120>  Novel formulations for antibodies

<130>  HEX-013 EP

<160>  17

<170>  PatentIn version 3.5

<210>  1
<211>  20
<212>  PRT
<213>  Homo sapiens

<400>  1

Gly Asn Val Phe Ser Cys Ser Val Met His Glu Ala Leu His Asn His
1               5                   10                  15

Tyr Thr Gln Lys
            20

<210>  2
<211>  11
<212>  PRT
<213>  Homo sapiens

<400>  2

Arg Ala Ser Gln Ser Val Ser Ser Tyr Leu Ala
1               5                   10

<210>  3
<211>  7
<212>  PRT
<213>  Homo sapiens

<400>  3

Asp Ala Ser Asn Arg Ala Thr
1               5

<210>  4
<211>  9
<212>  PRT
<213>  Homo sapiens

<400>  4

Gln Gln Ser Ser Asn Trp Pro Arg Thr
1               5

<210>  5
<211>  5
<212>  PRT

<213>  Homo sapiens

<400>  5

Asn Ser Gly Met His
1               5


<210>  6
<211>  17
<212>  PRT
<213>  Homo Sapiens

<400>  6

Val Ile Trp Tyr Asp Gly Ser Lys Arg Tyr Tyr Ala Asp Ser Val Lys
1               5                   10                  15


Gly


<210>  7
<211>  4
<212>  PRT
<213>  Homo sapiens

<400>  7

Asn Asp Asp Tyr
1


<210>  8
<211>  214
<212>  PRT
<213>  Homno sapiens

<400>  8

Glu Ile Val Leu Thr Gln Ser Pro Ala Thr Leu Ser Leu Ser Pro Gly
1               5                   10                  15


Glu Arg Ala Thr Leu Ser Cys Arg Ala Ser Gln Ser Val Ser Ser Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 9
<211> 439
<212> PRT
<213> Homo sapiens

<400> 9

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr

340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly
        435

<210>  10
<211>  15
<212>  PRT
<213>  mus msuculus

<400>  10

Arg Ala Ser Lys Gly Val Ser Thr Ser Gly Tyr Ser Tyr Leu His
1               5                   10                  15

<210>  11
<211>  7
<212>  PRT
<213>  mus musculus

<400>  11

Leu Ala Ser Tyr Leu Glu Ser
1               5

<210>  12
<211>  9
<212>  PRT
<213>  mus msuculus

<400>  12

Gln His Ser Arg Asp Leu Pro Leu Thr
1               5

<210>  13
<211>  5
<212>  PRT

<213>  mus musculus

<400>  13

Asn Tyr Tyr Met Tyr
1               5

<210>  14
<211>  17
<212>  PRT
<213>  mus musculus

<400>  14

Gly Ile Asn Pro Ser Asn Gly Gly Thr Asn Phe Asn Glu Lys Phe Lys
1               5                   10                  15

Asn

<210>  15
<211>  11
<212>  PRT
<213>  mus musculus

<400>  15

Arg Asp Tyr Arg Phe Asp Met Gly Phe Asp Tyr
1               5                   10

<210>  16
<211>  218
<212>  PRT
<213>  Artificial sequence

<220>
<223>  Humanized mouse antibody light chain

<400>  16

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  17
<211>  447
<212>  PRT
<213>  Artificial sequence

<220>
<223>  Humanized mouse antibody heavy chain

<400>  17

Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

## Description

The present invention relates to a stable, preferably liquid, pharmaceutical formulation comprising an antibody, a buffer, a surfactant, a stabilizer selected from the group consisting of sugars and sugar alcohols, and further optionally a chelator and/or oxygen-scavenger. Furthermore, the present invention relates to an article of manufacture comprising a container and a stable pharmaceutical formulation of the invention

Advances in biotechnology have made it possible to produce a variety of proteins for pharmaceutical applications. Because proteins are large and complex (i.e., possessing multiple functional groups in addition to complex three-dimensional structures), the formulation of such proteins poses special problems. For a protein to remain biologically active, a formulation must preserve the conformational integrity of at least a core sequence of the protein's amino acids, while at the same time protecting the protein's multiple functional groups from degradation. Proteins may suffer from a lack of stability, and monoclonal and polyclonal antibodies in particular may be relatively unstable.

Among the many problems in technical development of biotherapeutical antibodies, chemical modifications during storage pose a key challenge. Specifically, oxidation of methionine in biotherapeutical drug substances such as antibodies is of great concern since it may alter critical properties as efficacy (e.g. reduced target binding of an antibody), or safety (e.g. greater immunogenic potential due to increased aggregation). Antibodies are exposed to oxidative stress due to oxygen present in the headspace, metal ion leachables stemming from the primary packaging material or processing materials, or from trace impurities of excipients used in the formulation. Frequently, marked oxidation behaviour of antibodies can be observed. Oxidation of antibodies in turn may correlate with increased aggregation rates during storage in solution. This correlation highlights the need to establish tight control of oxidation in antibody formulations in order to prevent deterioration of key quality attributes of any given antibody drug product.

Although there are various antibody formulations on the market, there is still an urgent need to develop formulations, which give rise to steady, therapeutically effective blood levels of the antibody formulations over an extended period of time in a stable and convenient form.
The problem to be solved by the present invention was thus to provide further antibody formulations with satisfactory stability, in particular in terms of low oxidation and/or aggregation rates.

This problem is solved by the subject-matter as set forth below and in the appended claims.

In a first aspect the present invention relates to a stable, preferably liquid, pharmaceutical formulation comprising an antibody, a buffer, a surfactant, a stabilizer selected from the group of sugars and sugar alcohols, in particular sorbitol, and, optionally, a chelator and/or oxygen-scavenger.

A "stable" formulation according to the present invention is a formulation in which the antibody retains its physical stability and/or chemical stability and/or biological activity upon storage. Preferably, the antibody essentially retains its physical and chemical stability, as well as its biological activity upon storage. The storage period is generally selected based on the intended shelf-life of the formulation. Various analytical techniques for measuring protein stability are available in the art. Stability can be evaluated qualitatively and/or quantitatively in a variety of different ways, including evaluation of aggregate formation (for example using size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact antibody; peptide map (for example tryptic or LYS-C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomeriation), clipping/hydrolysis/fragmentation (e.g. hinge region fragmentation), succinimide formation, unpaired cysteine(s), N-terminal extension, C-terminal processing, glycosylation differences, etc. According to the present invention, a stable formulation is in particular a formulation which shows a low or negligible increase in methionine oxidation at surrogate site methionine within sequence GNVFSCSVMHEALHNHYTQK (which can be found in all IgG subtypes, SEQ ID NO:1). A low or negligible increase is an increase of less than 0.50% per month, preferably less than 0.20% per month, more preferably less than 0.15% per month, even more preferably less than 0.10% per month, after storage at 40°C for 12 weeks. Alternatively, or in addition, the stable formulation of the invention exhibits an increase in high molecular weight species as determined by size exclusion chromatography of less than 1.3% per month, more preferably of less than 1.2% per month, more preferably of less than 1.1% per month, more preferably of less than 1.0% per month, more preferably of less than 0.8% per month, even more preferably less than 0.6% per month, and most preferably of less than 0.4% per month after storage at 40°C for 12 weeks. Exemplary and preferred methods of assessing stability in terms of increase in methionine oxidation and increase in high molecular weight species are disclosed in the examples section of this application.

The antibody contained in the inventive formulation can be any antibody of interest. In certain embodiments, the antibody is a monoclonal antibody. In certain embodiments, the monoclonal antibody is a full-length antibody (e.g., IgG1, IgG4, etc.). Most preferably, the antibody is an IgG4 antibody. In certain embodiments, the monoclonal antibody is an antibody fragment (e.g., comprising an antigen binding region). For example, the antibody fragment is a Fab or F(ab')₂ fragment. In certain embodiments, the monoclonal antibody is a humanized antibody. In certain embodiments, the monoclonal antibody is susceptible to aggregation. The antibody may for example be directed against the following: tumour antigen CA-125, TNF-α, CD52, Aβ, CD25, B-lymphocyte stimulator (BLyS), vascular endothelial growth factor A (VEGF-A), IL-12, IL-23, IL-1β, epithelial cell adhesion molecule (EpCAM), epidermal growth factor receptor (EGFR; ErbB-1; HER1 in humans), RANK ligand (RANKL), CD11a, CD33, programmed cell death protein 1 (PD-1), carbonic anhydrase IX (CA-IX), CD20, cytotoxic T-lymphocyte-associated protein 4 (CTLA-4), CD23, IL-5, RSV glycoprotein F, CD3, fungal Hsp90, alpha-4 integrin, IgE Fc region, receptor tyrosine-protein kinase erbB-2 (HER2), or IL-6 receptor. In a preferred embodiment, the antibody comprises a light chain variable region comprising a complementarity determining region 1 (CDR1) according to SEQ ID NO:2, a CDR2 according to SEQ ID NO:3, and a CDR3 according to SEQ ID NO:4, and/or comprises a heavy chain variable region comprising a CDR1 according to SEQ ID NO:5, a CDR2 comprising according to SEQ ID NO:6, and a CDR3 comprising according to SEQ ID NO:7. Preferably, and antibody comprising these CDRs according to SEQ ID NO: 2 to 7 is an antibody which comprises a light chain according to SEQ ID NO:8 and a heavy chain according to SEQ ID NO:9. In an equally preferred embodiment, the antibody comprises a light chain variable region comprising a complementarity determining region 1 (CDR1) according to SEQ ID NO: 10, a CDR2 according to SEQ ID NO:11, and a CDR3 according to SEQ ID NO:12, and/or comprises a heavy chain variable region comprising a CDR1 according to SEQ ID NO:13, a CDR2 comprising according to SEQ ID NO:14, and a CDR3 comprising according to SEQ ID NO:15. Preferably, and antibody comprising these CDRs according to SEQ ID NO: 10 to 15 is an antibody comprising a light chain according to SEQ ID NO:16 and a heavy chain according to SEQ ID NO:17.

The antibody is preferably present in a concentration of up to about 80 mg/ml, preferably in the range of about 5 to about 50 mg/ml, more preferably in the range of 5 to 30 mg/ml, even more preferably in the range of about 10 mg/ml to 25 mg/ml. A particularly preferred concentration for an antibody comprising CDRs according to SEQ ID NO: 2 to 7, such as an antibody comprising a light chain according to SEQ ID NO:8 and a heavy chain according to SEQ ID NO:9 is about 10 mg/ml. A particularly preferred concentration for an antibody comprising CDRs according to SEQ ID NO: 10 to 15, such as an antibody comprising a light chain according to SEQ ID NO:16 and a heavy chain according to SEQ ID NO:17 is about 25 mg/ml.

The inventive stable antibody formulation comprises a buffer. The buffer can be any buffer suitable for storage of the antibody contained in the formulation. Preferably the buffer is selected from the group consisting of an acetate buffer, adipate buffer, histidine buffer, citrate buffer or succinate buffer. An "acetate buffer" is a buffer comprising acetate ions. Preferably, an acetate buffer does not comprise adipate, histidine, citrate and/or succinate ions. An example of an acetate buffer is a sodium acetate buffer. The acetate buffer has preferably a pH between about pH 5.0 to 6.5, preferably about pH 5.5. An "adipate buffer" is a buffer comprising adipate ions. Preferably, an adipate buffer does not comprise acetate, histidine, citrate and/or succinate ions. Examples of adipate buffers include sodium adipate, ammonium adipate, calcium adipate, magnesium adipate and potassium adipate solutions, with sodium adipate being particularly preferred. The adipate buffer has preferably a pH between about pH 5.0 to 6.5, about pH 5.5 to 6.2, or about pH 5.5 or about pH 6.0. A "histidine buffer" is a buffer comprising histidine ions. Preferably, a histidine buffer does not comprise acetate, adipate, citrate and/or succinate ions. Examples of histidine buffers include histidine chloride, histidine acetate, histidine phosphate, and histidine sulfate solutions, with histidine chloride (His/HCl) being particularly preferred. The histidine buffer or histidine-HCl buffer has preferably a pH between about pH 5.0 to 6.5, about pH 5.5 to 6.2, or about pH 6.0. A "citrate buffer" is a buffer comprising citrate ions. Preferably, a citrate buffer does not comprise acetate, adipate, histidine and/or succinate ions. Examples of citrate buffers include sodium citrate, ammonium citrate, calcium citrate, magnesium citrate and potassium citrate solutions. The citrate buffer has preferably a pH between about pH 5.0 to 6.5, about pH 5.5 to 6.2, or about pH 6.0. A "succinate buffer" is a buffer comprising succinate ions. Preferably, a succinate buffer does not comprise acetate, adipate, histidine and/or citrate ions. Examples of succinate buffers include sodium succinate, ammonium succinate, calcium succinate, magnesium succinate and potassium succinate solutions. The succinate buffer has preferably a pH between about pH 5.0 to 6.5, about pH 5.5 to 6.2, or about pH or about pH 6.0. In preferred embodiments of the invention, the buffer of the formulation of the present invention is a sodium succinate buffer (preferably at pH 6.0), a histidine-HCl buffer (preferably at pH 6.0) or a sodium adipate buffer (preferably at pH 6.0). pH is measured using standard glass bulb pH meter. Unless otherwise indicated, the pH values and ranges defined herein for formulations of the present invention reflect the pH at the respective storage temperature of the formulation, which is in the range of 2-8°C. In buffers comprising an antibody comprising CDRs according to SEQ ID NO: 2 to 7, such as an antibody comprising a light chain according to SEQ ID NO:8 and a heavy chain according to SEQ ID NO:9, the buffer has preferably a pH of 6.0. In buffers comprising an antibody comprising CDRs according to SEQ ID NO: 10 to 15, such as an antibody comprising a light chain according to SEQ ID NO:16 and a heavy chain according to SEQ ID NO:17, the buffer has preferably a pH of 5.5. In preferred embodiments, in particular for formulations comprising an antibody comprising CDRs according to SEQ ID NO: 2 to 7, such as an antibody comprising a light chain according to SEQ ID NO:8 and a heavy chain according to SEQ ID NO:9, the buffer is selected from the group consisting of adipate buffer, histidine buffer, and succinate buffer. In other preferred embodiments, in particular for formulations comprising an antibody comprising CDRs according to SEQ ID NO: 10 to 15, such as an antibody comprising a light chain according to SEQ ID NO:16 and a heavy chain according to SEQ ID NO:17, the buffer is selected from the group consisting of acetate buffer, adipate buffer, and succinate buffer. The concentration of the buffer in the inventive formulation is preferably in the range of 5 to 50 mM, even more preferably in the range of 10 to 20 mM. Most preferably, the concentration of the buffer is 10 mM. Preferred buffers for the formulation of the present invention, in particular for formulations comprising an antibody comprising CDRs according to SEQ ID NO: 2 to 7, such as an antibody comprising a light chain according to SEQ ID NO:8 and a heavy chain according to SEQ ID NO:9, are a 20 mM sodium succinate buffer, a 10 mM histidine/HCl buffer as well as a 10 mM sodium adipate buffer. Other preferred buffers for the formulation of the present invention, in particular for formulations comprising an antibody comprising CDRs according to SEQ ID NO: 10 to 15, such as an antibody comprising a light chain according to SEQ ID NO:16 and a heavy chain according to SEQ ID NO:17, are a 10 mM succinate buffer, a 10 mM acetate buffer as well as a 10 mM adipate buffer.

Aside of the buffer, the inventive formulation also comprises a surfactant. The surfactant can be in principle any (non-ionic) surfactant suitable for antibody formulation, but it is preferably, selected from the group of polysorbate 20, polysorbate 80, a poloxamer (such as poloxamer 188) and combinations thereof. Most preferably, the surfactant is polysorbate 20 or polysorbate 80. Typically, but not necessarily, the surfactant, and in particular polysorbate 20 or polysorbate 80, will be present in a concentration of about 0.01% to about 0.1% w/v. Formulations of the present invention may for example comprise about 0.1 mg/ml, about 0.2 mg/ml, about 0.3 mg/ml, about 0.4 mg/ml, about 0.5 mg/ml, about 0.6 mg/ml, about 0.7 mg/ml, about 0.8 mg/ml, about 0.9 mg/ml or about 1 mg/ml. In preferred embodiments of the invention, the surfactant is chosen from polysorbate 20 and polysorbate 80, and is present in a concentration of about 0.1 mg/ml to about 0.3 mg/ml. More preferably, the concentration of polysorbate 20 and polysorbate 80 is about 0.2 mg/ml. Most preferably, the surfactant is polysorbate 80 in a concentration of about 0.2 mg/ml.

A further component of the inventive formulation is a stabilizer selected from the group of sugars and sugar alcohols. Examples of sugars are lactose, fructose, maltose, trehalose and sucrose. Most preferably, the sugar is sucrose. Preferred examples of sugar alcohols are arabitol, mannitol and sorbitol. Most preferably, in particular if the formulation of the invention is a liquid formulation, sorbitol is a component of the inventive formulation. Sorbitol can help to suppress for example antibody aggregation, in particular in the course of freeze/thaw cycles. Typically, sorbitol is present in a concentration no greater than approximately 1 M in the liquid formulation of the invention. In some embodiments, sorbitol is present in a concentration no greater than approximately 300 mM. In many embodiments, the concentration of sorbitol will be in the range of 100 to 350 mM, more preferably in the range of 150 to 300 mM, even more preferably in the range of 165 mM to 285 mM sorbitol. Preferred sorbitol concentrations are for example 165 mM, 215 mM, 265 mM and 285 mM sorbitol. In some embodiments of the invention, the stable (preferably liquid) formulation of the invention does not contain mannitol. If the inventive formulation contains sucrose, it is preferably used in the range of 100 to 300 mM sucrose, preferably in the range of 140 to 210 mM sucrose. The formulation may also comprise a combination of sucrose and 2-hydroxypropyl-beta-cyclodextrin. In some cases, in particular if the inventive formulation comprises an antibody comprising CDRs according to SEQ ID NO: 10 to 15, such as an antibody comprising a light chain according to SEQ ID NO: 16 and a heavy chain according to SEQ ID NO:17, the formulation may comprise a sugar or a sugar alcohol, such as sorbitol or a combination of sucrose and 2-hydroxypropyl-beta-cyclodextrin.

A further, optional, component of the inventive formulation is a chelator and/or oxygen-scavenger or combinations of both. While said component is optional, it is nonetheless preferred if said optional component is present in the inventive formulation. Typical chelators are citrate, diethylenetriaminepentaacetic acid (DTPA), and ethylenediaminetetraacetic acid (EDTA), with the latter two being more preferred in the context of the present invention, and EDTA being the most preferred chelator of the invention. Examples for oxygen scavengers are methionine, sodium thiosulfate, catalase, or platinum, wherein methionine is the preferred oxygen scavenger for the present invention. Moreover, it is preferred if the inventive formulation comprises only one of chelator and oxygen-scavenger, not both. In case the inventive formulation comprises a chelator, such as DTPA or EDTA, the such chelator may be present in a concentration in the range of 5 to 150 µM, preferably in the range of 20 to 100 µM. Particularly preferred concentrations are 20 µM, 50µM, and 100 µM. Most preferred is a chelator concentration of 20 µM. In case the inventive formulation comprises an oxygen scavenger such as methionine, then such scavenger is preferably present in a concentration of about 100 µM to 10 mM. A particularly preferred concentration of methionine is about 6702 µM. Instead of using a chelator/and or oxygen scavenger, one may also consider to flush the headspace of the container containing the inventive formulation with an inert gas, e.g. with nitrogen, in order to displace oxygen and to minimize oxidation.

Finally, the inventive formulation may optionally also comprise salt such as sodium sulphate or sodium chloride or combinations thereof. Sodium chloride can reduce the viscosity of a formulation. The inventive formulation may - without being limited thereto - comprise 10 to 100 mM salt, for example 10 to 20 mM sodium sulphate, in particular 16 mM sodium sulphate, or may comprise 25 to 75 mM sodium chloride, in particular 40 to 60 mM sodium chloride, in particular 50 mM sodium chloride. Using a salt is in particular an option in cases where the inventive formulation comprises an antibody comprising CDRs according to SEQ ID NO: 2 to 7, such as an antibody comprising a light chain according to SEQ ID NO:8 and a heavy chain according to SEQ ID NO:9.

The stable formulation according to the present invention is preferably a liquid formulation. However, in some embodiments, in particular where the formulation does not comprise sorbitol, (but instead, for example, a sugar such as sucrose or a combination of sucrose and 2-hydroxypropyl-beta-cyclodextrin), the formulation may also be present in lyophilized form.

Liquid formulations specifically contemplated by the inventors of the present application, in particular in cases where the inventive formulation comprises an antibody comprising CDRs according to SEQ ID NO: 2 to 7, such as an antibody comprising a light chain according to SEQ ID NO:8 and a heavy chain according to SEQ ID NO:9 comprise a) a succinate buffer, EDTA, sorbitol and polysorbate 80, b) a histidine buffer, sodium chloride, EDTA, sorbitol and polysorbate 20, or c) a sodium adipate buffer, methionine, sorbitol and polysorbate 80. For instance, inventive formulations may comprise a) 20 mM sodium succinate buffer, pH 6.0, 265 mM sorbitol, 20 µM or 50 µM EDTA, and 0.2 mg/ml polysorbate 80, and no salt, i.e. sodium chloride and/or sodium sulphate, in particular no sodium chloride, b) 10 mM histidine/HCl buffer, pH 6.0, 50 mM sodium chloride, 215 mM sorbitol, 20 µM or 50 µM EDTA, and 0.2 mg/ml polysorbate 20, c) 10 mM histidine/HCl buffer, pH 6.0, 50 mM sodium chloride, 165 mM sorbitol, 20 µM or 50 µM EDTA, and 0.2 mg/ml polysorbate 20, or d) 10 mM sodium adipate buffer, pH 6.0, 265 mM or 285 mM sorbitol, 6702 µM methionine and 0.2 mg/ml polysorbate 80, and also no salt, i.e. no sodium chloride and/or sodium sulphate, in particular no sodium chloride.

In other embodiments, in particular in cases where the inventive formulation comprises an antibody comprising CDRs according to SEQ ID NO: 10 to 15, such as an antibody comprising a light chain according to SEQ ID NO: 16 and a heavy chain according to SEQ ID NO: 17, the inventors specifically contemplate the following buffers: a) a succinate buffer, sucrose and polysorbate 80, b) an acetate buffer, sucrose and polysorbate 80, and c) an adipate buffer, sucrose and polysorbate 80 (the sucrose in these formulations can be replaced by sorbitol). For instance, such formulations may comprise a) a 10 mM succinate buffer, 204.5 mM sucrose and 0.2 mg/ml polysorbate 80, b) a 10 mM acetate buffer, 204,5 mM sucrose and 0.2 mg/ml polysorbate 80, c) a 10 mM adipate buffer, 204,5 mM sucrose and 0.2 mg/ml polysorbate 80 or d) 10 mM succinate buffer, 204,5 mM sorbitol and 0.2 mg/ml polysorbate 80. In some of these embodiments, the formulation may comprise a combination of sucrose and 2 hydroxypropyl-beta-cyclodextrin (e.g. Kleptose^{®}), e.g. about 146 mM sucrose and about 22 mM 2 hydroxypropyl-beta-cyclodextrin or about 160 mM sucrose and about 40 mM 2 hydroxypropyl-beta-cyclodextrin.

In a further aspect, the present invention relates to an article of manufacture comprising a container, a stable, preferably liquid, pharmaceutical formulation according to the present invention and instructions for its use. The article of manufacture may in particular be a container such as a pre-filled syringe, such as a filled glass syringe, or a glass vial.

Finally, the present invention relates to a stable pharmaceutical formulation according to the present invention, in particular a stable liquid pharmaceutical formulation according to the present invention, for use in a method of treating a disease of the human or animal body, in particular wherein the disease is cancer. The cancer may be any type of cancer for which the antibody of the inventive formulation is therapeutically effective. For instance, the cancer may be selected from the group consisting of lung, colon, bladder, cervix, skin, ovary, endometrial, gastric, head and neck, breast, melanoma, pancreas, kidney, prostate, oesophagus, stomach or bile duct cancer. Similarly, the cancer may for example be selected from the group consisting of renal carcinoma (RCC), classical Hodgkin lymphoma (cHL), non-Hodgkin's lymphoma (NHL), non-small cell lung carcinoma (NSCLC), squamous cell cancer of the head and neck (SCCHN or HNSCC, respectively), urothelial carcinoma, glioma, colorectal cancer, chronic lymphocytic leukaemia (CLL), follicular lymphoma, and breast carcinoma.

The term "comprising", as used herein, shall not be construed as being limited to the meaning "consisting of" (i.e. excluding the presence of additional other matter). Rather, "comprising" implies that optionally additional matter, features or steps may be present. The term "comprising" encompasses as particularly envisioned embodiments falling within its scope "consisting of" (i.e. excluding the presence of additional other matter) and "comprising but not consisting of" (i.e. requiring the presence of additional other matter, features or steps), with the former being more preferred.

The use of the word "a" or "an", when used herein, may mean "one," but it is also consistent with the meaning of "one or more," "at least one," and "one or more than one."

### Figures

In the following a brief description of the appended figures will be given. The figures are intended to illustrate the present invention in more detail. However, it is not intended to limit the scope of the invention to only these specifically illustrated embodiments.
- Fig. 1:: illustrates the methionine oxidation rate per month for an antibody heavy chain (SEQ ID NO:9); total incubation time: 12 weeks at 40°C. Error bars indicate standard errors of linear regression for 4 data points.
- Fig. 2:: illustrates the formation of high molecular weight species of an antibody comprising an antibody heavy chain according to SEQ ID NO:9 and a light chain according to SEQ ID NO:8); per month of incubation at 40°C. Total incubation time 12 weeks for 40°C. Antibody as determined by size exclusion chromatography. Error bars indicate fit error from linear regression of 4 data points.
- Fig. 3:: illustrates the increase of aggregates over 3 months storage at 40 °C in different formulations for an antibody comprising an antibody heavy chain according to SEQ ID NO:17 and a light chain according to SEQ ID NO: 16.
- Fig. 4:: illustrates the increase of acidic species over 3 months storage at 40 °C in different formulations for an antibody comprising an antibody heavy chain according to SEQ ID NO:17 and a light chain according to SEQ ID NO: 16.
- Fig. 5:: illustrates the increase of degradation products over 3 months storage at 40 °C in different formulations for an antibody comprising an antibody heavy chain according to SEQ ID NO:17 and a light chain according to SEQ ID NO: 16.

### Examples

In the following, specific examples illustrating various embodiments and aspects of the invention are presented. However, the present invention shall not to be limited in scope by the specific embodiments described herein. Indeed, various modifications of the invention in addition to those described herein will become readily apparent to those skilled in the art from the foregoing description, accompanying figures and the examples below. All such modifications fall within the scope of the appended claims.

### Example 1: Analysis of oxidation of an antibody in the presence of DTPA, EDTA or oxygen scavenger methionine

The effect of various formulations on methionine oxidation rate per month of an antibody heavy chain (SEQ ID NO:9) after storage for 12 weeks at a storage temperature of 40°C was tested. For this purpose, the following experimental set-up was used:
10 mL of sterile filtered sample solution was filled into 25R glass vials (hydrolytic glass type I) and closed with a FluroTec(R) coated stopper and a crimp cap. Filled vials were stored inverted and incubated until the respective pull points at 40 ± 2°C / 75 ± 5 % RH. Oxidation was determined using a reduced peptide mapping approach. Samples were denatured using guanidinium hydrochloride, followed by reduction with dithiothreitol and alkylation using iodoacetamide. Peptide fragments were generated using lysyl-endopeptidase C (Lys-C) and subsequently analysed using RP-HPLC FLD. Relative quantification was performed on one methionine sulfoxide containing reporter peptide derived from the IgG C-terminus (Met 421) as percentage of the sum of its native peptide and the oxidised variant. Note that methionine oxidation results in formation of methionine sulfoxide diastereomeres, resulting in two potential reporter peptides. Here, only one of them was chosen as reporter peptide for quantification of methionine oxidation.

Formulations to be analysed were selected from the following list:

**Table 1 List of formulations, all formulations contain protein at 10 mg / mL**

| **Formulation ID** | **Buffer type** | **Buffer pH** | **Buffer conc. (mM** | **Salt** | **Sugar alcohol** | **Chelator** | **Surfactant** |
|---|---|---|---|---|---|---|---|
| F01 | Sodium Citrate | 6.0 | 20.0 | 50 mM NaCl | 165 mM mannitol | 20 µM DTPA | 0.2 mg/mL PS80 |
| F02 | Sodium Citrate | 6.0 | 10.0 | 16 mM Na₂SO₄ | 165 mM sorbitol | 50 µM EDTA | 0.2 mg/mL PS80 |
| F03 | Sodium Succinate | 6.0 | 20.0 | 50 mM NaCl | 165 mM sorbitol | 20 µM DTPA | 0.2 mg/mL PS80 |
| F04 | Sodium Succinate | 6.0 | 20.0 | 50 mM NaCl | 165 mM sorbitol | 50 µM EDTA | 0.2 mg/mL PS80 |
| F05 | Sodium Succinate | 6.0 | 20.0 | 50 mM NaCl | 165 mM sorbitol | None | 0.2 mg/mL PS80 |
| F06a | Sodium Succinate | 6.0 | 20.0 | none | 265 mM sorbitol | 20 µM EDTA | 0.2 mg/mL PS80 |
| F06 | Sodium Succinate | 6.0 | 20.0 | none | 265 mM sorbitol | 50 µM EDTA | 0.2 mg/mL PS80 |
| F07 | Sodium Succinate | 6.0 | 20.0 | 50 mM NaCl | 165 mM sorbitol | 6702 µM methionine | 0.2 mg/mL PS80 |
| F08 | His/HCl | 6.0 | 10.0 | 50 mM NaCl | 165 mM sorbitol | 20 µM DTPA | 0.2 mg/mL PS80 |
| F09 | His/HCl | 6.0 | 10.0 | 50 mM NaCl | 165 mM sorbitol | 100 µM EDTA | 0.2 mg/mL PS80 |
| F10a | His/HCl | 6.0 | 10.0 | 50 mM NaCl | 215 mM sorbitol | 20 µM EDTA | 0.2 mg/mL PS20 |
| F10 | His/HCl | 6.0 | 10.0 | 50 mM NaCl | 165 mM sorbitol | 50 µM EDTA | 0.2 mg/mL PS20 |
| F11 | His/HCl | 6.0 | 10.0 | 50 mM NaCl | 165 mM sorbitol | 20 µM EDTA | 0.2 mg/mL PS80 |
| F12 | His/HCl | 6.0 | 10.0 | 50 mM NaCl | 165 mM sorbitol | None | 0.2 mg/mL PS80 |
| F13 | His/HCl | 6.0 | 10.0 | 50 mM NaCl | 165 mM sorbitol | 6702 µM methionine | 0.2 mg/mL PS80 |
| F14 | His/HCl | 6.0 | 10.0 | None | 265 mM sorbitol | 6702 µM methionine | 0.2 mg/mL PS80 |
| F15 | Sodium adipate | 6.0 | 20.0 | 50 mM NaCl | 165 mM sorbitol | 50 µM EDTA | 0.2 mg/mL PS80 |
| F16a | Sodium adipate | 6.0 | 10.0 | None | 285 mM sorbitol | 6702 µM methionine | 0.2 mg/mL PS80 |
| F16 | Sodium adipate | 6.0 | 10.0 | None | 265 mM sorbitol | 6702 µM methionine | 0.2 mg/mL PS80 |

| | | | | | | | |
|---|---|---|---|---|---|---|---|
| PS 80 = polysorbate 80 | | | | | | | |

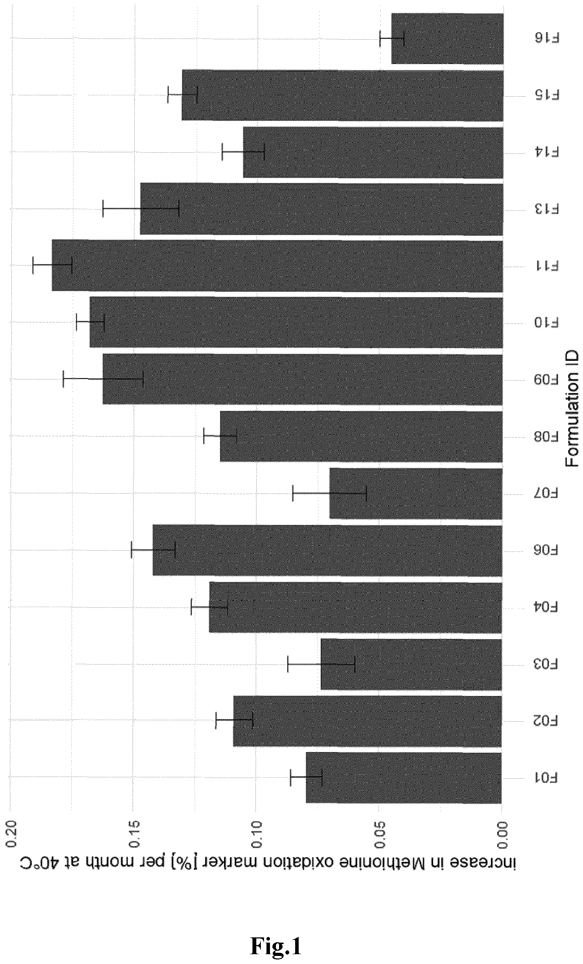

Results of this experiment are provided in Fig. 1. The inventors found that the chelators EDTA and DTPA, as well as the oxidation-scavenger methionine are effective in preventing antibody oxidation, as indicated by low oxidation rates. Furthermore, methionine, followed by DTPA, proved especially useful in protecting the antibody from oxidation in the concentration ranges used. Moreover, methionine countered oxidation strongest with diprotic acids as buffers, such as succinic acid or adipic acid.

### Example 2: Analysis of formation of high molecular weight species per month of incubation

In a further series of experiments, formulations of table 1 above (comprising 10 mg/ml of an antibody comprising a light chain according to SEQ ID NO:8 and a heavy chain according to SEQ ID NO:9) were analyzed with respect to their impact on aggregation, i.e. formation of high molecular weight species per month after storage at a storage temperature of 40°C for 12 weeks.

Samples were prepared and stored as in Example 1. High Molecular Weight species (HMWs) were determined using SEC-HPLC UV at sample concentrations of 1 mg/mL.

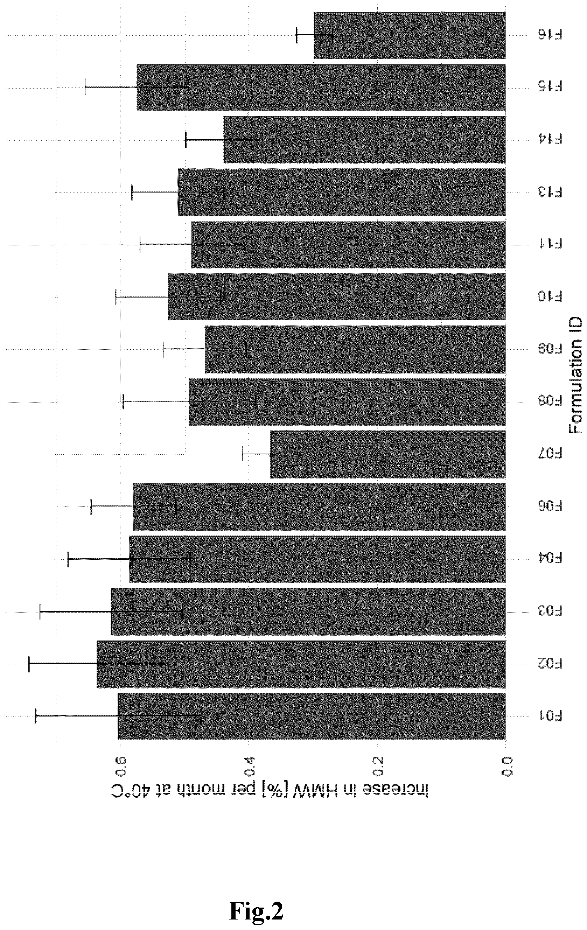

The result of this experiment is shown in Figure 2. In particular, the data show that the antibody showed good aggregation behaviour in the tested formulations. A particularly superior aggregation behaviour was apparent when the antibody was formulated in a diprotic acid buffer (such as succinic acid, adipic acid) and/or with methionine.

### Example 3: Analysis of further antibody

In a further series of experiments, a different antibody and different formulations were tested (light chain according to SEQ ID NO:16; heavy chain according to SEQ ID NO:17. Formulations to be analysed were selected from the following list:

**Table 2 List of formulations, all formulations contain protein at 25 mg / mL**

| **Formulation ID** | **Buffer type** | **Buffer pH** | **Buffer conc. (mM** | **Tonifier/Stabilizer** | **Surfactant** |
|---|---|---|---|---|---|
| F1 | Histidine | 5.5 | 10 | 204.5 mM sucrose | 0.2 mg/mL PS80 |
| F2 | Succinate | 5.5 | 10 | 204.5 mM sucrose | 0.2 mg/mL PS80 |
| F3 | Acetate | 5.5 | 10 | 204.5 mM sucrose | 0.2 mg/mL PS80 |
| F4 | Adipate | 5.5 | 10 | 204.5 mM sucrose | 0.2 mg/mL PS80 |
| F5 | Succinate | 5.5 | 10 | 160 mM sucrose/40 mM 2-hydroxypr opyl-beta-cyclodextrin | 0.2 mg/mL PS80 |
| F5a | Succinate | 5.5 | 10 | 146 mM sucrose/22 mM 2-hydroxypr opyl-beta-cyclodextrin | 0.2 mg/mL PS80 |
| F6 | Succinate | 5.5 | 10 | 204.5 mM sorbitol | 0.2 mg/mL PS80 |

| | | | | | |
|---|---|---|---|---|---|
| PS 80 = polysorbate 80 | | | | | |

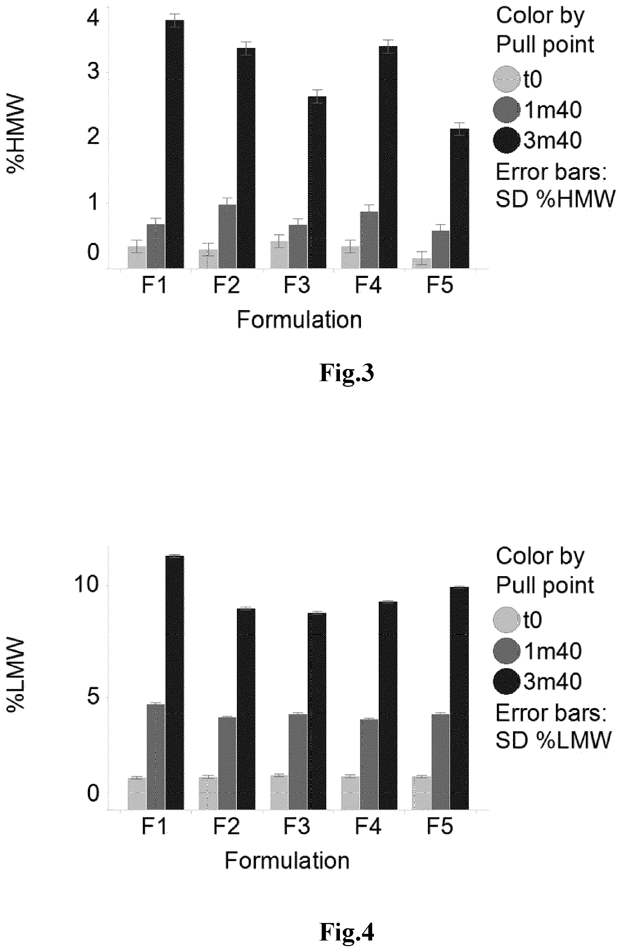

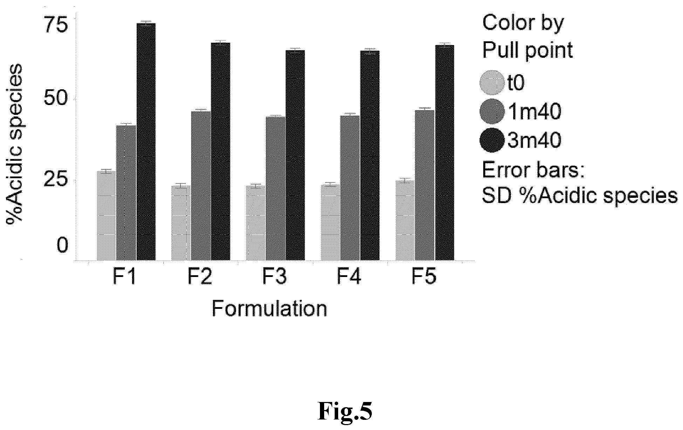

Compounding of samples was performed on Tecan liquid handling system. After sterile filtration, 150 µL of each formulation (F1 - F5) was placed to a storage temperature of 40 °C in 96 matrix - well type I glass tubes for up to 3 months. Aggregation was evaluated by UPLC SEC. Degradation products were monitored by non-reduced CE-SDS and change in charge variants was evaluated by ICE. Results of experiments are provided in Figures 3 - 5 and table 3 below.

**Table 3: SEC, nr CE - SDS and iCE data for t0, 1 month and 3 months 40 °C.**

| **Formulation** | **Pull point** | **Description** | **pH** | **SEC %AP** | **nr CE-SDS %LMW** | **% iCE Acidic** |
|---|---|---|---|---|---|---|
| **F1** | t0 | 25 mg/mL protein, 10 mM His/HCl buffer, 204.5 mM Sucrose, 0.2 mg/mL PS80 | 5.5 | 0.3 | 1.4 | 27.7 |
| **F2** | t0 | 25 mg/mL protein, 10 mM Succinate buffer, 204.5 mM Sucrose, 0.2 mg/mL PS80 | 5.5 | 0.3 | 1.5 | 23.2 |
| **F3** | t0 | 25 mg/mL protein, 10 mM Acetate buffer, 204.5 mM Sucrose, 0.2 mg/mL PS80 | 5.4 | 0.4 | 1.5 | 23.1 |
| **F4** | t0 | 25 mg/mL protein, 10 mM Adipate buffer, 204.5 mM Sucrose, 0.2 mg/mL PS80 | 5.5 | 0.3 | 1.5 | 23.6 |
| **F5** | t0 | 25 mg/mL protein, 10 mM Acetate buffer, 160 mM Sucrose/40 mM 2 hydroxypropyl-beta-cyclodextrin, 0.2 mg/mL PS80 | 5.5 | 0.2 | 1.5 | 24.8 |
| **F1** | 1Mo 40°C | 25 mg/mL protein, 10 mM His/HCl buffer, 204.5 mM Sucrose, 0.2 mg/mL PS80 | 5.5 | 0.7 | 4.7 | 41.9 |
| **F2** | 1Mo 40°C | 25 mg/mL protein, 10 mM Succinate buffer, 204.5 mM Sucrose, 0.2 mg/mL PS80 | 5.5 | 1.0 | 4.1 | 46.1 |
| **F3** | 1Mo 40°C | 25 mg/mL protein, 10 mM Acetate buffer, 204.5 mM Sucrose, 0.2 mg/mL PS80 | 5.4 | 0.7 | 4.3 | 44.4 |
| **F4** | 1Mo 40°C | 25 mg/mL protein, 10 mM Adipate buffer, 204.5 mM Sucrose, 0.2 mg/mL PS80 | 5.5 | 0.9 | 4.0 | 44.9 |
| **F5** | 1Mo 40°C | 25 mg/mL protein, 10 mM Acetate buffer, 160 mM Sucrose/40 mM 2 hydroxypropyl-beta-cyclodextrin, 0.2 mg/mL PS80 | 5.5 | 0.6 | 4.3 | 46.5 |
| **F1** | 3Mo 40°C | 25 mg/mL protein, 10 mM His/HCl buffer, 204.5 mM Sucrose, 0.2 mg/mL PS80 | 5.5 | 3.8 | 11.3 | 73.4 |
| **F2** | 3Mo 40°C | 25 mg/mL protein, 10 mM Succinate buffer, 204.5 mM Sucrose, 0.2 mg/mL PS80 | 5.5 | 3.4 | 9.0 | 67.4 |
| **F3** | 3Mo 40°C | 25 mg/mL protein, 10 mM Acetate buffer, 204.5 mM Sucrose, 0.2 mg/mL PS80 | 5.4 | 2.6 | 8.8 | 65.0 |
| **F4** | 3Mo 40°C | 25 mg/mL protein, 10 mM Adipate buffer, 204.5 mM Sucrose, 0.2 mg/mL PS80 | 5.5 | 3.4 | 9.3 | 64.8 |
| **F5** | 3Mo 40°C | 25 mg/mL protein, 10 mM Acetate buffer, 160 mM Sucrose/40 mM 2 hydroxypropyl-beta-cyclodextrin, 0.2 mg/mL PS80 | 5.5 | 2.1 | 9.9 | 66.7 |

## Claims

1. A stable, preferably liquid, pharmaceutical formulation comprising an IgG4 antibody, a buffer, a surfactant, a stabilizer selected from the group consisting of sugars and sugar alcohols, and further optionally a chelator and/or oxygen-scavenger.

2. The liquid formulation according to claim 1, wherein the formulation is a liquid formulation and wherein the formulation comprises sorbitol and/or sucrose.

3. The stable formulation according to claim 1 or 2, wherein the buffer is an acetate buffer, adipate buffer, histidine buffer, citrate buffer or succinate buffer, preferably an adipate buffer, acetate buffer, histidine buffer, or succinate buffer.

4. The stable formulation according to any one of the preceding claims, wherein the buffer has a pH of 5 to 6, preferably of 5.5 or 6.0.

5. The stable formulation according to any one of the preceding claims, wherein the surfactant is selected from polysorbate 20, polysorbate 80, a poloxamer and combinations thereof, in particular wherein the surfactant is polysorbate 20 or polysorbate 80.

6. The stable formulation according to any one of the preceding claims, wherein the formulation comprises a chelator and/or oxygen-scavenger and the chelator/oxygen-scavenger is selected from the group consisting of diethylenetriaminepentaacetic acid (DTPA), ethylenediaminetetraacetic acid (EDTA), methionine or combinations thereof, preferably wherein the chelator is EDTA and the oxygen-scavenger is methionine.

7. The stable, formulation according to any one of the preceding claims, wherein the composition comprises a salt, in particular sodium chloride.

8. The stable formulation according to any one of the preceding claims, wherein the composition comprises antibody in a concentration in the range of about 5 to 50 mg/ml, preferably in in a concentration of about 10 mg/ml or about 25 mg/ml.

9. The stable formulation according to any one of the preceding claims, wherein the formulation comprises:
a) a succinate buffer, EDTA, sorbitol and polysorbate 80,
b) a histidine buffer, sodium chloride, EDTA, sorbitol and polysorbate 20,
c) a sodium adipate buffer, methionine, sorbitol and polysorbate 80,
d) a succinate buffer, sucrose and polysorbate 80,
e) an acetate buffer, sucrose and polysorbate 80, or
f) an adipate buffer, sucrose and polysorbate 80.

10. The stable formulation according to any one of claims 1 to 9, wherein the formulation comprises:
a) a 20 mM sodium succinate buffer, pH 6.0, 265 mM sorbitol, 20 µM or 50 µM EDTA, and 0.2 mg/ml polysorbate 80, and no sodium chloride and/or sodium sulphate,
b) a 10 mM histidine/HCl buffer, pH 6.0, 50 mM sodium chloride, 215 mM sorbitol, 20 µM or 50 µM EDTA, and 0.2 mg/ml polysorbate 20,
c) a 10 mM histidine/HCl buffer, pH 6.0, 50 mM sodium chloride, 165 mM sorbitol, 20 µM or 50 µM EDTA, and 0.2 mg/ml polysorbate 20,
d) a 10 mM sodium adipate buffer, pH 6.0, 265 mM or 285 mM sorbitol, 6702 µM methionine and 0.2 mg/ml polysorbate 80,
e) a 10 mM adipate buffer, pH 5.5, 204.5 mM sucrose, and 0.2 mg/ml polysorbate 80 and optionally a chelator and/or oxygen-scavenger,
f) a 10 mM acetate buffer, pH 5.5, 204.5 mM sucrose, and 0.2 mg/ml polysorbate 80, and optionally a chelator and/or oxygen-scavenger, or
g) a 10 mM succinate buffer, pH 5.5, 204.5 mM sucrose, and 0.2 mg/ml polysorbate 80, and optionally a chelator and/or oxygen-scavenger.

11. The stable formulation according to claim 9 or 10, wherein the formulation comprises 2 hydroxypropyl-beta-cyclodextrin, in particular wherein the formulation comprises a succinate or adipate or acetate buffer and/or sucrose and 2 hydroxypropyl-beta-cyclodextrin.

12. An article of manufacture comprising a container, a stable pharmaceutical formulation of any one of claims 1 to 11, and instructions for its use.

13. The article of claim 12, wherein the article is container, in particular a pre-filled syringe or glass vial.

14. The stable liquid pharmaceutical formulation of any one of claims 1 to 11 for use in a method of treating a disease of the human or animal body.
